Europäisches Patentamt

(19) European Patent Office  (11) Veröffentlichungsnummer: **0 144 489**

Office européen des brevets  **B1**

(12) **EUROPÄISCHE PATENTSCHRIFT**

(45) Veröffentlichungstag der Patentschrift: 15.02.89

(21) Anmeldenummer: 84104156.9

(22) Anmeldetag: 12.04.84

(51) Int. Cl.⁴: **A 61 K 37/38,** A 61 K 9/08, A 61 K 9/48 // (A61K37/38, 31:195)

(54) Verwendung eines zerebral und peripher wirkenden Antihypoxidotikums.

(30) Priorität: 28.04.83 CH 2374/83

(43) Veröffentlichungstag der Anmeldung: 19.06.85 Patentblatt 85/25

(45) Bekanntmachung des Hinweises auf die Patenterteilung: 15.02.89 Patentblatt 89/7

(84) Benannte Vertragsstaaten: AT BE CH DE FR GB LI NL

(56) Entgegenhaltungen: UNLISTED DRUGS, Band 22, Nr. 5, Mai 1970, Seite 69, Absatz f, Chatham, N.J., US; "Choritropin"

Die Akte enthält technische Angaben, die nach dem Eingang der Anmeldung eingereicht wurden und die nicht in dieser Patentschrift enthalten sind.

(73) Patentinhaber: Eurich, Rolf, Dr., Alte Dieburger Strasse 38, D-6109 Mühltal- Trautheim (DE)

(72) Erfinder: Eurich, Rolf, Dr., Alte Dieburger Strasse 38, D-6109 Mühltal- Trautheim (DE)

(74) Vertreter: Zutter, Hans Johann Niklaus, EPROVA AG Forschungsinstitut Im Laternenacker 5, CH-8200 Schaffhausen (CH)

EP 0 144 489 B1

LIBER, STOCKHOLM 1989

**Beschreibung**

Die vorliegende Erfindung betrifft die Verwendung eines zentral und peripher wirkenden Antihypoxidotikums auf der Basis einer Kombination von Choriongonadotropin und natürlichen Aminosäuren, insbesondere zur Behandlung von arteriosklerotisch bedingten Schädigungen.

Mit dem Älterwerden der Menschen nehmen auch deren Risikofaktoren zu. Dazu gesellen sich auch zunehmende Schwierigkeiten für die persönliche, familiäre und gesellschaftliche Situation dieser Menschen. Zerebrale Mangeldurchblutungen schädigen das Hirngewebe, vermindern die Gewebsatmung und führen zu Erscheinungen, welche gewöhnlich mehr oder weniger korrekt als Arteriosklerose bezeichnet werden. Der konservativen Therapie der zerebrovaskulären Insuffizienz kommt daher eine grosse Bedeutung zu, wobei der soziale Aspekt bei den betroffenen Menschen von eminenter Bedeutung ist.

Die Aufrechterhaltung einer menschenwürdigen Lebensform des zerebral Geschädigten stellt eine grosse Verpflichtung dar, unabhängig davon, ob der Patient zuhause oder in einem Pflegeheim untergebracht ist.

Die durch zerebrale Minderdurchblutungen verursachten Hirnschädigungen bilden die grösste Krankheitsgruppe in der Neuropsychiatrie. Sie sind bedingt durch die Arteriosklerose der Hirngefässe aber auch durch einen reduzierten Metabolismus der Gehirnzellen im allgemeinen. Infolge ihrer meist generalisierten Ausbreitung zeichnen sich die Folgen dieser Hirnschädigungen vornehmlich durch eine psychiatrische Symptomatik aus. Diese ist gekennzeichnet durch eine Einengung der affektiven Reaktion und Anpassung, durch Störungen der Auffassung, der Konzentration und Ausdauer, durch raschere Ermüdbarkeit, Tendenz zu Verstimmungen und Affektreaktionen. Ausserdem finden sich Beeinträchtigung von Merkfähigkeit und Gedächtnis, Antriebsschwäche, flüchtige oder länger anhaltende Verwirrtheitszustände und verschieden stark ausgeprägte Grade von Bewusstseinstrübung hin bis zur Bewusstlosigkeit und Koma.

In den letzten Jahrzehnten hat man versucht, durch Anwendung von Vasodilatoren eine Verbesserung der Hirndurchblutung und eine Erhöung des Perfusionsvolumens zu erreichen. Die damit erzielten Ergebnisse waren nicht durchwegs befriedigend. Das rührt wahrscheinlich daher, dass der geschädigte zerebrale Zellstoffwechsel durch die bessere Blutversorgung und die damit erreichte erhöhte Glukosezufuhr nicht grundsätzlich verbessert werden konnte.

Heute steht die Normalisierung des zerebralen Zellstoffwechsels im Vordergrund der Therapie der zerebrovaskulären Insuffizienz. Man setzt dazu immer mehr sogenannte Antihypoxidotika ein. Das sind Pharmaka, welche die infolge Sauerstoffmangels herabgesetzte Zellatmung fördern. Sie beseitigen die Störungen der oxidativen Energiegewinnung in den Zellen. Diese Pharmaka sollen eine Verbesserung des Zellstoffwechsels und gleichzeitig eine Verbesserung der zerebralen Hämodynamik bewirken. Wenn es dadurch gelingt, Störungen des Energiestoffwechsels des Zentralnervensystems und der damit eingehenden funktionellen Ausfälle zu beseitigen oder zu mildern, so ist das Ziel der Therapie erreicht worden.

Als Antihypoxidotika wurden zwei Gruppen von Präparaten vorgeschlagen:

1.  Präparate, welche den Zellstoffwechsel verbessern sollen wie Solcoseryl® und Hydergin®.
    Solcoseryl® ist ein Dialysekonzentrat aus deproteinisiertem, enzymatisch angedautem Kälberblut nach DE-PS-1 076 888.
    Hydergin® ist ein Kombinationspräparat enthaltend Dihydroergocornin, Dihydroergocristin, Dihydro-α-ergokryptin sowie Dihydro-β-ergokryptin.
    Es liegen noch wenig schlüssige Untersuchungen über die antihypoxidotische Wirkung dieser Präparate vor.
2.  Präparate, welche die kollaterale zerebrale Zirkulation verbessern, wie beispielsweise die unter dem internationalen Freinamen (INN) verwendeten Arzneimittel Hexobendin (= 3,4,5-Trimethoxybenzoesäure-1,2-ethandiylbis[(methylimino)-3,1-propandiyl]-ester, Pyritinol (= 3,3'-[Dithiobis(methylen)]bis[5-hydroxy-6-methyl-4-pyridinmethanol]) und Betahistidinhydrochlorid (= N-Methyl-2-pyridinethanamin dihydrochlorid). Die Präparate dieser 2. Gruppe wirken nur indirekt antihypoxidotisch.

Es wurde überraschend gefunden, dass die Kombination von menschlichem Choriongonadotropin (HCG) mit natürlichen Aminosäuren ausgezeichnete antihypoxidotische Aktivitäten aufweist.

Menschliches Choriongonadotropin wird in Dosen von 250 bis 5'000 internationalen Einheiten verwendet zur Behandlung der Amenorrhoe und Sterilität bei der Frau sowie bei Kryptorchismus, Hypogonadismus, Pubertas tarda und Impotentia coeundi. Rote Liste <u>1980</u>: 49031 Primogonyl®.

Natürliche Aminosäuren, wie z. B. mono-Magnesium-L-aspartat werden angewandt bei Störungen der Coronardurchblutung und des Myokardstoffwechsels, etwa bei Angina pectoris. Rote Liste <u>1980</u>: 61072 Magnesiocard®.

Mono-Magnesium-L-glutamat wird zur Beseitigung von Magnesiummangel während der Schwangerschaft, zur Therapie der Präeklampsie, Eklampsie bei Wadenkrämpfen und stenokardischen Beschwerden verwendet. Rote Liste <u>1980</u>: 61073, Magnesium Verla®.

Im europäischen Recherchenbericht wird das Präparat Choritropin, enthaltend Choriongonadotropin, Thiamin und L-Glutaminsäure, zitiert [Unlisted Drugs <u>22</u> (5) 69 (1970)]. Es ist zur Behandlung von Hypogonadismus, Menstruationsbeschwerden und Beschwerden vor der Geburt indiziert. Diese Verwendung stimmt im wesentlichen überein mit derjenigen von Primogonyl®, das nur aus Choriongonadotropin besteht

2

und kein Thiamin und keine Aminosäuren enthält.

Gegenstand der Erfindung ist daher die Verwendung einer Kombination aus 50 bis 1'500 internationalen Einheiten von menschlichem Choriongonadotropin (HCG) pro Verabreichungsdosis und natürlichen Aminosäuren zur Herstellung eines Antihypoxidotikums zur Behandlung von zerebralen und peripheren Durchblutungs- und Stoffwechselstörungen.

Als natürliche Aminosäuren enthält dieses Antihypoxidotikum L-Glutaminsäure, L-Isoleucin und L-Alanin oder Salze davon. Es kann aber ausserdem noch weitere Aminosäuren wie beispielsweise γ-Aminobuttersäure, Leucin oder Asparaginsäure bzw. Salze davon enthalten.

Als Salze der Aminosäuren werden Kalium-, Magnesium- oder Natrium-Salze verwendet.

Die Verwendung der genannten Kombination ist im speziellen dadurch gekennzeichnet, dass sie ausser menschlichem Choriongonadotropin pro Dosis noch

50 bis 500mg L-Glutaminsäure,
10 bis 150 mg L-Isoleucin und
10 bis 150 mg L-Alanin bzw. Kalium- oder Magnesiumsalze davon enthält.

Das Antihypoxidotikum kann in Form von intramuskulär zu verabreichenden Injektionslösungen als magensaftresistente Kapseln oder Dragees oder in Form von Suppositorien verabreicht werden.

Das Verfahren zur Herstellung dis Antihypoxidotikums zur Behandlung von zerebralen peripheren Durchblutungs- und Stoffwechselstörungen, das selbst nicht Gegenstand der Erfindung ist, ist dadurch gekennzeichnet, dass man pro Verabreichungsdosis 50 bis 500 mg L-Glutaminsäure, 10 bis 150 mg L-Isoleucin und 10 bis 150 mg L-Alanin oder Kalium- bzw. Magnesiumsalze dieser Aminosäuren in wenig Wasser auflöst, den pH der Lösung auf 6,6 bis 7,6 einstellt und der erhaltenen Lösung noch 50 bis 1'500 internationale Einheiten von menschlichem Choriongonadotropin zusetzt.

Die erhaltene Lösung wird beispielsweise sterilfiltriert und anschliessend lyophilisiert, um sie in eine lagerfähige Form überzuführen. Unmittelbar vor dem Gebrauch wird das Lyophilisat durch Zusatz von 1 bis 20 ml bidestilliertem Wasser oder sterilisierter physiologischer Kochsalzlösung in die gebrauchsfertige Injektionslösung übergeführt.

Man kann aber vorteilhafterweise auch die Lösung der Aminosäuren bzw. deren Salze sterilisieren und unmittelbar vor Gebrauch in eine Trockenampulle, welche die gewünschte Menge (50 - 1'500 I.E.) Choriongonadotropin enthält, einspritzen und derart die gebrauchsfertige Injektionslösung erzeugen.

Zur Herstellung von festen Darreichungsformen des Antihypoxidotikums werden 50 bis 500 mg L-Glutaminsäure, 10 bis 150 mg L-Isoleucin und 10 bis 150 mg L-Alanin oder die äquivalente Menge Kalium- oder Magnesiumsalz davon sowie 50 bis 1'500 internationale Einheiten von menschlichem Choriongonadotropin zusammen mit Schutzkolloiden vermischt und in magensaftresistente Kapseln abgefüllt zu einer Tablette gepresst und magensaftresistent dragiert oder zu einem Suppositorium verarbeitet.

Die pharmakologische Wirkung des neuen Antihypoxidotikums lässt sich beispielsweise durch die Bestimmung des DNS-Gehaltes verschiedener Organe sowie deren [35]S-Sulfat-Inkorporationsraten unter Einwirkung des Präparates nachweisen und messen.

Der interzellulär liegenden Grundsubstanz (Interzellularsubstanz) kommt eine wichtige Funktion im interzellulären Stofftransport sowie im Transport zwischen Blutgefäss-System und Zellen zu. Eine fördernde oder hemmende Wirkung auf den Leistungsstoffwechsel der Interzellularsubstanz verschiedener Organe bzw. Gewebe lässt daher eine Aussage über die Wirkung von Pharmaka zu.

Der Zellgehalt der Ratten-Aorta, hier biochemisch durch den DNS-Gehalt bestimmt, fällt bis zum 12. Lebensmonat kontinuierlich ab und bleibt dann bis ins hohe Senium konstant. Während am Rippenknorpel eine Erhöhung der [35]S-Sulfat-Inkorporation zwischen dem 2. und 3. Lebensjahr der Ratte zu beobachten ist, zeigt die Ratten-Aorta einen Abfall der [35]S-Sulfat-Inkorporation, bezogen auf den Zellgehalt, bis ins hohe Senium der Ratte, die Rückenhaut der Ratte dagegen nicht. Dies bedeutet, dass die Syntheseleistung der Haut, im Gegensatz zu Knorpel und Aorta, offenbar bei alten und jungen Ratten gleich bleibt. Beim Menschen ist ein entsprechender Reifungs- und Alterungsverlauf speziell an der Aorta nachgewiesen (Lindner J. et al., Verh. Dtsch. Ges. Path. 51, 228-236 (1967)).

Es wurde gefunden, dass der Leistungsstoffwechsel des Herzens, der Aorta und der Rückenhaut der Ratte durch das neue Antihypoxidotikum entscheidend verbessert wird. Dies zeigt sich in der positiven Beeinflussung des DNS-Gehaltes sowie der [35]S-Sulfat-Inkorporationsraten der untersuchten Organe.

Tierexperimentelle Untersuchungen:
Die Untersuchungen wurden an 108 weiblichen Sprague-Dawley-Ratten durchgeführt, die bei Versuchsbeginn 15 Monate alt waren. Die Applikation der untersuchten Substanzen erfolgte täglich peroral mittels Schlundsonde bzw. durch intramuskuläre Injektion. Die Tiere wurden vor Versuchsbeginn randomisiert auf die einzelnen Versuchsgruppen aufgeteilt. Die Körpergewichte wurden wöchentlich bestimmt.
Kontrollgruppe:
Die Tiere blieben unbehandelt.
TAA-Gruppe: Die Tiere erhielten 25 mg Thioacetamid pro kg Körpergewicht im Tag per os.
Präparate-Gruppe: Die Tiere erhielten täglich das Präparat bestehend aus 150 internationalen Einheiten HCG, 50 mg L-Isoleucin, 50 mg L-Alanin und 200 mg L-Glutaminsäure pro kg Körpergewicht intramuskulär verabreicht.

TAA + Präparat-Gruppe: Diese Tiergruppe erhielt die oben angegebene Tagesdosis TAA und Präparat.

Pen-Gruppe: Diese Rattengruppe erhielt täglich 200 mg D-Penicillamin pro kg Körpergewicht und Tag per os.

Erläuterung:

Thioacetamid (TAA) ist ein Zellgift, das den Leistungsstoffwechsel der Zelle reduziert, der sinkende DNS-Gehalt des Gewebes ist ein Mass für den Abfall des Leistungsstoffwechsels der Zelle. Vergl. dazu M. Helle et al und Fahrhat et al, Arzneimittel-Forschung 29 (1979) S. 71-81 und S. 82-90.

D-Penicillamin (Pen) wird hier als Vergleichspräparat verwendet. Es wirkt der TAA-Schädigung entgegen.

Die DNS-Bestimmung im Herz erfolgte nach der Methode von Dische 1929, adaptiert von Seibert 1940, bei den Aorten nach der Methode von Burton 1956. Siehe beispielsweise Mulzer et al, Arzneimittel-Forschung 29 (1979) S. 207-212.

Die Ergebnisse der Tierversuche sind in den Tabellen 1, 2, 3 und 4 aufgeführt.

Aus den Messdaten der Tierversuche geht hervor, dass unter Einwirkung des erfindungsgemässen Präparates der DNS-Gehalt und die [35] S-Sulfat-Inkorporationsraten des Herzens, der Rückenhaut und der Aorta signifikant (pc 0.01) erhöht wurde.

Das Präparat vermag auch den durch Thioacetamid (TAA) bewirkten Abfall des Leistungsstoffwechsels der Zelle zu kompensieren und zwar stärker als D-Penicillamin (Pen).

Das erfindungsgemässe antihypoxidotisch wirkende Präparat ist praktisch untoxisch. Nach subkutaner Applikation von bis zu 2'500 mg pro kg Körpergewicht ist keine der damit behandelten Ratten gestorben.

**Tab. 1:** DNS-Gehalt des Herzens ($\mu$g/g TG) ($\bar{x} \pm$ s) (n = 6)

| Substanz | Behandlungszeit: | | |
| --- | --- | --- | --- |
| | 4 Wochen | 8 Wochen | 12 Wochen |
| Kontrolle | 656,21 ± 87,27 | 693,84 ± 68,33 | 663,46 ± 72,43 |
| TAA | 488,75 ± 48,45 | 519,75 ± 79,86 | 497,25 ± 39,79 |
| Präparat | 732,38 ± 39,75 | 768,17 ± 54,78 | 709,12 ± 47,41 |
| TAA + Präparat | 676,46 ± 26,28 | 714,62 ± 48,13 | 683,68 ± 34,27 |
| Pen | 783,87 ± 67,65 | 792,61 ± 69,19 | 758,30 ± 59,32 |
| TAA + Pen | 635,74 ± 98,27 | 661,46 ± 84,12 | 684,15 ± 64,37 |

**Tab. 2:** DNS-Gehalt der Aorta ($\mu$g/g TG) ($\bar{x} \pm$ s) (n = 6)

| Substanz Behandlungszeit: | | | |
| --- | --- | --- | --- |
| | 4 Wochen | 8 Wochen | 12 Wochen |
| Kontrolle | 464,24 ± 82,15 | 392,65 ± 26,57 | 438,41 ± 47,36 |
| TAA | 388,18 ± 63,35 | 317,27 ± 37,84 | 337,67 ± 39,78 |
| Präparat | 508,34 ± 67,22 | 442,22 ± 88,37 | 468,88 ± 53,64 |
| TAA + Präparat | 425,87 ± 41,77 | 379,78 ± 48,76 | 389,23 ± 67,82 |
| Pen | 530,71 ± 53,43 | 449,09 ± 54,12 | 501,42 ± 72,61 |
| TAA + Pen | 478,72 ± 69,54 | 404,88 ± 61,72 | 452,07 ± 63,24 |

**Tab. 3:** DNS-Gehalt der Rückenhaut ($\mu$g/g TG) ($\bar{x} \pm$ s) (n = 6)

| Substanz | Behandlungszeit: | | |
| --- | --- | --- | --- |
| | 4 Wochen | 8 Wochen | 12 Wochen |
| Kontrolle | 433,61 ± 63,87 | 366,74 ± 47,36 | 409,41 ± 28,79 |
| TAA | 216,80 ± 54,12 | 305,74 ± 88,37 | 382,82 ± 53,64 |
| Präparat | 482,16 ± 43,27 | 409,53 ± 37,48 | 488,12 ± 39,78 |
| TAA + Präparat | 385,37 ± 67,12 | 349,75 ± 54,72 | 503,34 ± 67,82 |
| Pen | 412,87 ± 59,82 | 385,12 ± 61,75 | 477,37 ± 59,32 |
| TAA + Pen | 382,28 ± 68,45 | 328,14 ± 48,67 | 573,21 ± 67,14 |

**Tab. 4:** [35] S-Sulfat-Inkorporation (dpm/100 mg TG) ($\bar{x} \pm s$)

|  | Kontrolle | Präparat |
|---|---|---|
| Herz | 862,5 ± 112,7 | 1037,4 ± 98,9 |
| Aorta | 4203,8 ± 263,5 | 4738,7 ± 284,7 |
| Rückenhaut | 1768,2 ± 195,3 | 2044,15 ± 182,7 |

Klinische Untersuchungen:

Aus den tierexperimentellen Untersuchungen geht hervor, dass die Kombination von menschlichem Choriongonadotropin (HCG), L-Glutaminsäure, L-Isoleucin und L-Alanin bzw. deren Kalium- und Magnesiumsalzen bei der Ratte eine Verbesserung des Leistungsstoffwechsels der Zelle herbeiführen. Eine Pilotstudie an Patienten mit zerebrovaskulärer Insuffizenz und teilweise mit peripheren Durchblutungs- und Stoffwechselstörungen zeigte, dass eine auffallende Besserung mit dem neuen Antihypoxidotikum erzielt werden kann. Insbesondere wurde eine Verbesserung der Konzentrationsfähigkeit, des Kurzzeitgedächtnisses sowie eine beachtliche Zunahme der Leistungssteigerung und des Antriebes festgestellt.

Zur Behandlung und Vorbeugung krankhafter Abläufe der Biomorphose, insbesondere der Störungen des Energiestoffwechsels des Zentralnervensystems und der damit einhergehenden funktionellen Ausfälle wird das Präparat täglich oder mehrmals wöchentlich intramuskulär injiziert oder in Form von Kapseln oder magensaftresistenten Dragées oder Suppositorien verabreicht.

Für die klinischen Untersuchungen wurden 53 Patienten mit zerebrovaskulärer Insuffizienz ausgewählt. Es handelt sich durchwegs um prognostisch ungünstige Fälle. Das Durchschnittsalter betrug 81.6 Jahre. Als Basismedikation erhielten sämtliche Patienten das pharmakologisch geprüfte Präparat gemäss Beispiel 1 in den ersten beiden Behandlungswochen täglich. Ab der dritten Behandlungswoche dreimal wöchentlich. Die zur Beurteilung des Therapieerfolges ausgewählten Symptome und deren Beeinflussung sind in der Tabelle 5 dargestellt. Während des Einsatzes des Präparates wurde festgestellt, dass sich ein positiver Wirkungseintritt nach 5 bis 8 Injektionen einstellt und die Wirkung des Medikamentes nach Absetzen noch 2 bis 3 Wochen anhält. Die Behandlung wurde über einen Zeitraum von 12 Wochen durchgeführt. Unerwünschte Nebenwirkungen wurden nicht beobachtet. Im Vordergrund des Therapieerfolges standen eine Besserung der Konzentrationsfähigkeit, des Kurzzeitgedächtnisses, von Verwirrtheitszuständen und Desorientierung sowie eine beachtliche Zunahme der Leistungssteigerung und des Antriebes. Besonders beeindruckend war das Aufhellen des Sensoriums und das Nachlassen von über Wochen und Monate bestehenden Verwirrtheitszuständen, was vom Pflegepersonal und den Angehörigen besonders beobachtet und gemeldet wurde.

Während der Behandlung wurden regelmässige Laborkontrollen durchgeführt. Die Kontrolluntersuchungen liessen keine Abweichungen von der Norm erkennen.

Eindrucksvoll waren auch einige Fälle von Dekubitus, die zuvor jeglicher Behandlung getrotzt haben. Nach Verabreichung des erfindungsgemässen Präparates heilte der Dekubitus teilweise bereits innerhalb von 6 bis 8 Wochen völlig ab. Dies erlaubt den Schluss, dass das Präparat neben der zerebralen auch periphere Durchblutungs- und Stoffwechselstörungen zu beseitigen vermag.

**Tab. 5:** Beurteilung des Behandlungserfolges nach 12wöchiger Therapie

| Symptome |  | nach Therapie | | |
|---|---|---|---|---|
|  |  | gebessert | % | unverändert |
| Konzentrationsschwäche | 44 | 35 | 79,5 | 9 |
| Desorientiertheit | 25 | 22 | 88,0 | 3 |
| Verwirrtheit | 16 | 10 | 62,5 | 6 |
| Depressive Verstimmung | 23 | 19 | 82,6 | 4 |
| Reizbarkeit | 14 | 8 | 57,1 | 6 |
| Antriebsarmut | 36 | 27 | 75,0 | 9 |
| rasche Ermüdbarkeit | 36 | 30 | 83,3 | 6 |
| Dekubitus | 8 | 5 | 62,5 | 3 |
| Appetitlosigkeit | 34 | 34 | 100,0 | - |

Die nachfolgenden Beispiele betreffen die Herstellung von Darreichungsformen von zur erfindungsgemässen Verwendung als Antihypoxidotikum geeigneten Kombinationspräparaten aus Choriongonadotropin und natürlichen Aminosäuren.

**Beispiel 1**

Antihypoxidotikum enthaltend pro Einzeldpsis
150 Internationale Einheiten (I.E.) Choriongonadotropin (HCG)
200 mg L-Glutaminsäure
50 mg L-Isoleucin
50 mg L-Alanin

Diese Einzeldosis wird nach Auflösen in 3 ml physiologischer Kochsalzlösung intramuskulär verabreicht.

**Beispiel 2**

Antihypoxidotikum enthaltend pro Einzeldosis
50 mg L-Glutaminsäure
50 mg L-Asparaginsäure
50 mg γ-Aminobuttersäure
10 mg L-Leucin
10 mg L-Alanin
200 I.E. HCG

Diese Einzeldosis wird nach Auflösen in 2 ml physiologischer Kochsalzlösung intramuskulär verabreicht.

**Beispiel 3**

Antihypoxidotikum bestehend pro Einzeldosis aus
1) Lösung enthaltend:
    100 mg L-Glutaminsäure
    20 mg L-Isoleucin
    10 mg L-Alanin in
    2 ml bidestilliertem Wasser
2) 200 I.E. HCG in Serumfläschchen oder Trockenampulle.

**Beispiel 4**

Kapseln enthaltend pro Einzeldosis
100 mg Magnesiumglutaminat
50 mg Kalium-aspartat
50 mg Natrium-aspartat
20 mg L-Isoleucin
20 mg L-Alanin
300 I.E. HCG

abgefüllt in eine magensaftresistente Kapsel, welche vom Darmsaft aufgelöst wird.

**Beispiel 5**

Herstellung einer Darreichungsform aus Lyophilisat und physiologischer Kochsalzlösung.
10 g L-Glutaminsäure, 2 g L-Isoleucin sowie 2 g L-Alanin werden in 200 ml Wasser gelöst und durch Zusatz von wenig Natriumhydroxid auf pH 7 ± 0,3 eingestellt. Es wird so lange gerührt, bis völlige Lösung eingetreten ist. Nunmehr werden 10 mg eines standardisierten Lyophilisates, das pro mg 1000 I.E. menschliches Choriongondatropin enthält, der Lösung zugegeben und kurz durchgerührt, durch einen Filter mit einem Porendurchschnitt von 0,22 mμ filtriert und unter sterilen Bedingungen in Portionen von je 2 ml in Ampullen abgefüllt und anschliessend lyophilisiert. Die Ampullen werden zugeschmolzen. Unmittelbar vor Gebrauch wird das Lyophilisat mit jeweils 2,5 ml bidestilliertem Wasser versetzt, durch Schütteln in Lösung verbracht, mit der Injektionsspritze aufgezogen und intramuskulär verabreicht.

6

**Beispiel 6**

Herstellung einer Darreichungsform bestehen aus Solvens- und Trockenampulle.

1) Solvensampullen:

10 g L-Glutaminsäure, 2 g L-Isoleucin sowie 2 g L-Alanin werden in 200 ml Wasser gelöst und durch Zusatz von wenig Natriumhydroxid auf pH 7 ± 0,3 eingestellt. Es wird so lange gerührt, bis völlige Lösung eingetreten ist. Die Lösung wird durch einen Filter mit einem Porendurchmesser von 0,22 mµ filtriert und in Portionen von je 2 ml in Ampullen abgefüllt. Die Ampullen werden zugeschmolzen und in einem Autoklaven sterilisiert.

2) HCG-Trockenampullen:

Nunmehr werden 10 mg eines standardisierten Lyophilisates, das pro mg 1000 I.E. HCG enthält, in 200 ml Wasser pro inj. gelöst, kurz durchgerührt und durch einen Filter mit einem Porendurchmesser von 0,22 mµ filtriert und unter sterilen Bedingungen in Portionen von je 2 ml in Ampullen abgefüllt und das Wasser durch Gefriertrocknung entfernt. Die Ampullen werden zugeschmolzen. Unmittelbar vor Gebrauch wird die Trockenampulle mit der Solvensampulle versetzt, mit der Injektionsspritze aufgezogen und intramuskulär verabreicht.

**Beispiel 7**

Herstellung von Dragées.

10'000 g L-Glutaminsäure, 2000 g L-Isoleucin und 2000 g L-Alanin werden mit 2 l Stärkekleister, welcher 100 g Amylum solani enthält, in einer Knetmaschine angeteigt. 10 g eines standardisierten Lyophilisates, das pro mg 10'000 I.E. Choriongonadotropin enthält, werden in 500 ml ca. 30°C warmes Wasser eingetragen, bis zur völligen Auflösung gerührt, der feuchten Masse zugesetzt und in der Knetmaschine kräftig durchgerührt. Danach wird die feuchte Masse mit etwas trockener Stärke versetzt, in einer Granuliermaschine granuliert und im Vakuum getrocknet. Das fertige Granulat wird danach mit 865 g Amylum solani und 25 g Magnesiumstearat vermischt und zu Tabletten mit einem Kerngewicht von 0,15 g verpresst.

Der Wirkstoffgehalt pro Tablette beträgt 100 mg L-Glutaminsäure, 20 mg L-Isoleucin, 20 mg L-Alanin und 100 I.E. HCG.

Die Tabletten werden dragiert und mit einem magensaftresistenten Überzug versehen.

**Beispiel 8**

Herstellung von Kapseln.

Dieselben Wirkstoffe in denselben Mengen wie zur Herstellung von Dragées werden mit 1000 g Sojalecithin, 11500 g Rüböl gut durchgemischt. Diese Mischung wird in 100'000 darmlösliche Weichgelatine-Kapseln abgefüllt, deren Gelatinehülle zu 67 % aus Gelatine und 33 % aus Glycerin besteht.

**Beispiel 9**

Herstellung von Suppositorien.

10 g L-Glutaminsäure, 2 g L-Isoleucin sowie 2 g L-Alanin werden in 200 ml Wasser gelöst. Es wird so lange gerührt, bis völlige Lösung eingetreten ist. Nunmehr werden 10 mg eines Lyophilisates, das pro mg 1000 I.E. HCG enthält, der Lösung zugegeben und kurz durchgerührt.

Auf dem Wasserbad werden 2000 g Polyäthylenoxid erwärmt und mit der obigen Aminosäurelösung mit dem HCG unter gleichmässigem Rühren versetzt. Anschliessend wird die so gewonnene Masse in Suppositorien-Formen eingegossen. Nach dem Erkalten werden die Suppositorien kühl aufbewahrt.

**Patentansprüche**

1. Verwendung einer Kombination von 50 bis 1'500 internationalen Einheiten von menschlichem Choriongonadotropin (HCG) pro Verabreichungsdosis und natürlichen Aminosäuren zur Herstellung eines Antihypoxidotikums zur Behandlung von zerebralen und peripheren Durchblutungs- und Stoffwechselstörungen.

2. Verwendung einer Kombination nach Patentanspruch 1, dadurch gekennzeichnet, dass sie ausser Choriongonadotropin pro Dosis noch

7

50 bis 500 mg L-Glutaminsäure
10 bis 150 mg L-Isoleucin und
10 bis 150 mg L-Alanin
bzw. Salze davon enthält.

**Claims**

1. The use of a combination of 50 to 1,500 international units of human chorionic gonadotropin (HCG) per administration dose and natural amino acids for the preparation of an antihypoxidotic for the treatment of disturbances of cerebral and peripheral blood flow and metabolism.

2. The use of a combination according to patent Claim 1, characterized in that, apart from chorionic gonadotropin, it also contains per dose
50 to 500 mg of L-glutamic acid
10 to 150 mg of L-isoleucine and
10 to 150 mg of L-alanine
or salts thereof.

**Revendications**

1. Application d'une combinaison de 50 à 1 500 unités internationales de gonadotropine chorionique humaine (HCG) par dose d'administration et d'acides aminés naturels pour préparer un antihypoxydotique en vue du traitement des désordres de l'irrigation cérébrale et périphérique et du métabolisme.

2. Application d'une combinaison selon la revendication 1, caractérisée en ce qu'elle contient encore la gonadotropine chorionique, par dose
50 à 500 mg d'acide L-glutamique
10 à 150 mg de L-isoleucine et
10 à 150 mg de L-alanine
ou de leurs sels.